# EUROPÄISCHE PATENTSCHRIFT

(11) **EP 0 799 054 B1**
(45) Veröffentlichungstag und Bekanntmachung des Hinweises auf die Patenterteilung: **31.01.2001**
(21) Anmeldenummer: 95933300.6
(22) Anmeldetag: 26.09.1995
(51) Int. Cl.: A61K 41/00, A61K 51/08

(54) **KONJUGAT ZUR BEHANDLUNG VON ENTZÜNDLICHEN ERKRANKUNGEN**
CONJUGATE FOR TREATING INFLAMMATORY DISEASES
CONJUGUE POUR LE TRAITEMENT DE MALADIES INFLAMMATOIRES

(30) Priorität: 30.09.1994 DE 4435087
(43) Veröffentlichungstag der Anmeldung: 08.10.1997
(73) Patentinhaber: Deutsches Krebsforschungszentrum Stiftung des öffentlichen Rechts, 69120 Heidelberg (DE)
(72) Erfinder: SINN, Hansjörg, D-69118 Wiesloch (DE); SCHRENK, Hans-Hermann, D-67278 Zeiskam (DE); MAIER-BORST, Wolfgang, D-69221 Dossenheim (DE); STEHLE, Gerd, D-68305 Mannheim (DE); WUNDER, Andreas, D-69214 Eppelheim (DE); HOFF-BIEDERBECK, Dirk, D-6703 Ludwigshafen (DE); HEENE, Dieter, Ludwig, D-68259 Mannheim (DE)
(74) Vertreter: Schüssler, Andrea, Dr.
(86) Internationale Anmeldenummer: DE9501337
(87) Internationale Veröffentlichungsnummer: WO9610422

(56) Entgegenhaltungen:
- EP-A- 0 398 024
- WO-A-93/03035
- BE-A- 882 541
- DE-A- 4 017 439
- US-A- 4 466 951
- JOURNAL OF BIOLOGICAL CHEMISTRY, Bd. 263, Nr. 28, 5.Oktober 1988 MD US, Seiten 14122-14127, JANET L. MAXWELL ET AL. 'INULIN-125I-TYRAMINE, AN IMPROVED RESIUDALIZING LABEL FOR STUDIES ON SITES OF CATABOLISM OF CIRCULATING PROTEINS.'
- DATABASE CHEMABS CHEMICAL ABSTRACTS SERVICE, COLUMBUS, OHIO, US AN=119:198674, HAMBLIN, M. R. ET AL 'Conjugates between proteins and fluorescent dyes as potential photosensitizers' & INT. CONGR. SER. - EXCERPTA MED. (1992), 1011(PHOTODYNAMIC THERAPY AND BIOMEDICAL LASERS), 169-73 CODEN: EXMDA4;ISSN: 0531-5131, 1992
- EXPERIENTIA (1967), 23(11), 949-50 CODEN: EXPEAM, 1967 WINKELMAN, JAMES W. 'Metabolic studies on the accumulation of tetraphenylporphinesulfonate in tumors'

## Beschreibung

Die Erfindung betrifft die Verwendung von Konjugaten gemäß Anspruch 1, umfassend einen Wirkstoff, einen Linker und einen Träger, zur Behandlung und/oder Diagnose von entzündlichen Erkrankungen.

Zur Behandlung von entzündlichen Erkrankungen, wie Infektions- und/oder Autoimmun- und/oder Hauterkrankungen, werden bisher Arzneimittel verwendet, die in sehr hohen Dosen verabreicht werden müssen.Dies stellt eine große Belastung für die Leber dar. Desweiteren reichern sich diese Arzneimittel in vielen Geweben an, was eine weitere Belastung für den Körper darstellt.

Aus DE-A 39 12 792 und DE-A-40 17 439 sind Konjugate vorstehender Art bekannt, die zur Therapie und/oder Diagnose von Tumoren verwendet werden können.

Überraschenderweise hat sich nun gezeigt, daß sich diese Konjugate auch zur Behandlung und/oder Diagnose von entzündlichen Erkrankungen, wie Infektions- und/oder Autoimmun- und/oder Hauterkrankungen und/oder entzündlichen Neovaskularisationen, z.B. in der Hornhaut des Auges, eignen, ohne die Nachteile der bisher hierfür verwendeten Arzneimittel zu haben.

Erfindungsgemäß werden somit vorstehende Konjugate zur Behandlung und/oder Diagnose von entzündlichen Erkrankungen, wie Infektions- und/oder Autoimmun- und /oder Hauterkrankungen, wie Psoriasis, und/oder entzündlichen Neovaskularisationen, z.B. in der Hornhaut des Auges, verwendet.

Der Ausdruck "Wirkstoff" umfaßt Verbindungen jeglicher Art, die zur Therapie und/oder Diagnose von entzündlichen Erkrankungen, insbesondere Infektions- und/oder Autoimmun- und/oder Hauterkrankungen und/oder entzündlichen Neovaskularisationen verwendet werden können. Beispiele vorstehender Verbindungen sind radioaktiv markierte aromatische Verbindungen, photodynamisch-aktive Verbindungen und Chemotherapeutika. Beispiele photodynamischaktiver Verbindungen sind Porphyrin-Derivate, wie Tetracarboxyphenylporphyrin (TCPP) und Tetrahydroxyphenylporphyrin (THOPP), Chlorine und Bakteriochlorine. Beispiele der Chemotherapeutika sind Zytostatika und Antibiotika. Vertreter dieser sind z.B. Doxorubicin, Daunorubicin, Tetracyclin und Derivate davon sowie Antimetaboliten, wie Methotrexat. Vorstehende photodynamisch-aktive Verbindungen und Chemotherapeutika können auch markiert sein, z.B. mit einer radioaktiven Substanz, wie Jod. Desweiteren können von vorstehenden Verbindungen nicht nur eine einzelne, sondern auch mehrere in einem erfindungsgemäß verwendeten Konjugat vorliegen.

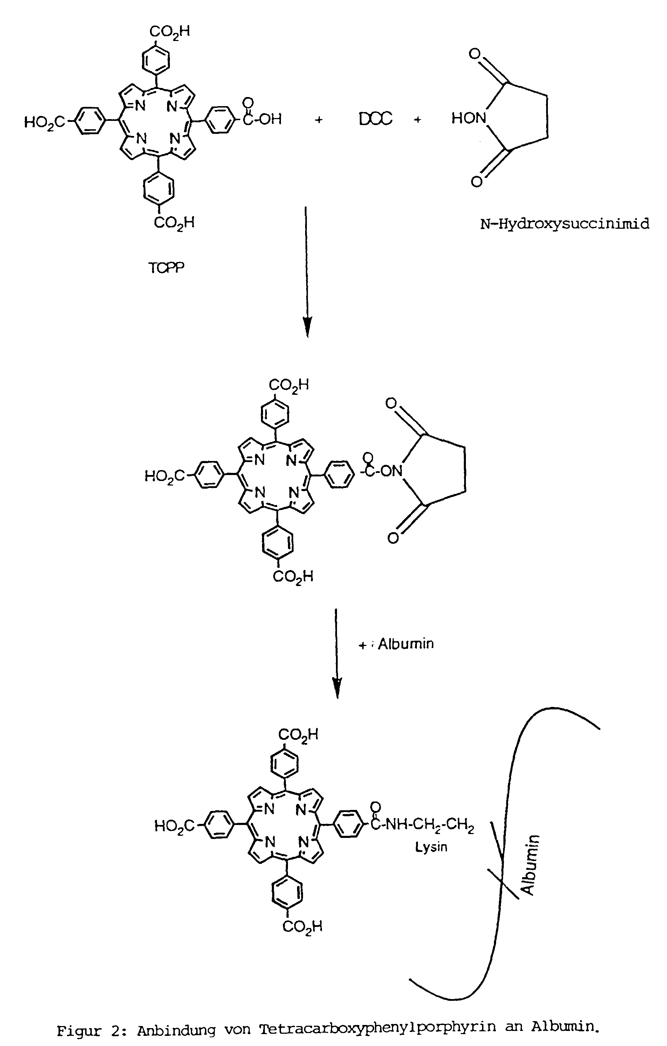

Der Ausdruck "Linker" umfaßt Verbindungen jeglicher Art, die zur Verknüpfung von zwei Komponenten des Konjugats, insbesondere von Protein und Wirkstoff, geeignet sind. Beispiele solcher Linker sind Cyanurchlorid und Derivate davon, die als Edukte zur Bildung des Konjugats eingesetzt werden. Ein Linker kann ferner bereits ein Teil des Wirkstoffs sein, so daß kein weiterer Linker in das Konjugat eingebracht werden muß. Ein Beispiel hierfür ist in Fig. 2 angegeben.

Der Ausdruck "Träger" umfaßt Albumin, Transferrin und/oder ein Polyethylenglykol. Vorzugsweise ist es Albumin, besonders bevorzugt in nativer Form, und Transferrin. Es können auch Proteinfragmente verwendet werden. Beispiele von Polyethylenglykolen sind solche mit einem Polymerisationsgrad n von 5 bis 250. Vorzugsweise sind die Polyethylenglykole an der endständigen Hydroxylgruppe mit einer C₁-C₁₂-Alkylgruppe, insbesondere Methylgruppe, verestert oder verethert. Ein mit einer Methylgruppe verethertes PEG wird als Methoxypolyethylenglykol (MPEG) bezeichnet.

In den Konjugaten können mehrere Polyethylenglykole vorliegen, die gleich oder verschieden sein können. Auch können ein oder mehrere Polyethylenglykole und ein Protein im Konjugat vorliegen.

Ein erfindungsgemäß verwendetes Konjugat kann auch einen Polyalkohol enthalten. Ein solcher kann vorzugsweise an den Wirkstoff gebunden sein. Der Ausdruck "Polyalkohol" umfaßt Atomgruppen jeglicher Art, die mindestens eine OH-Gruppe aufweisen und nicht als körperfremd angesehen werden. Die als Wirkstoff verwendeten Verbindungen können auch einen Polyalkohol im Molekül tragen. Es muß dann kein weiterer Polyalkohol mehr in das Konjugat eingebracht werden.

Beispiele des Polyalkohols weisen folgende Struktur auf: 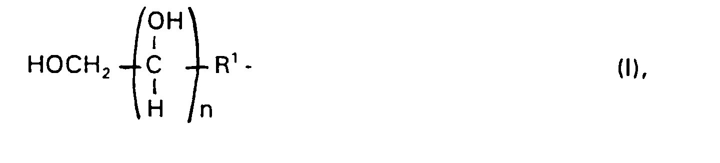 in der R¹ CHOH, CH₂, C=O oder CH₂NH ist und
n mindestens 1, vorzugsweise 1 bis 10 und am bevorzugtesten 3 bis 6 ist. Ein Beispiel eines solchen Polyalkohols ist ein Glucamin-Rest oder ein Derivat davon.

Weiterhin kann der Polyalkohol ein Tris(hydroxymethyl)-aminomethan-Rest oder ein Derivat davon sein.

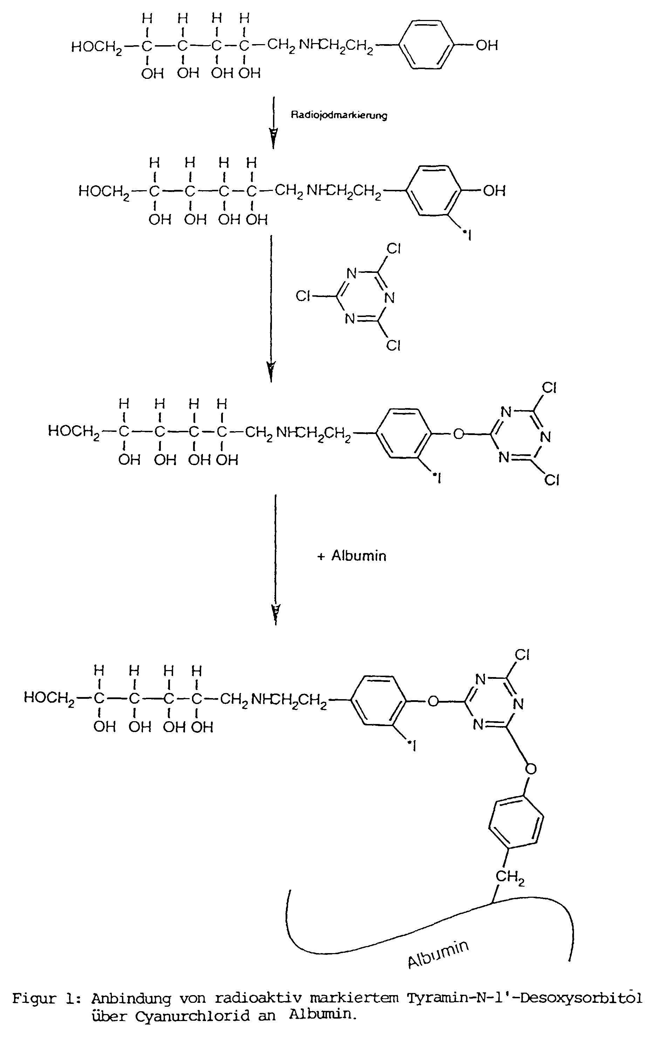

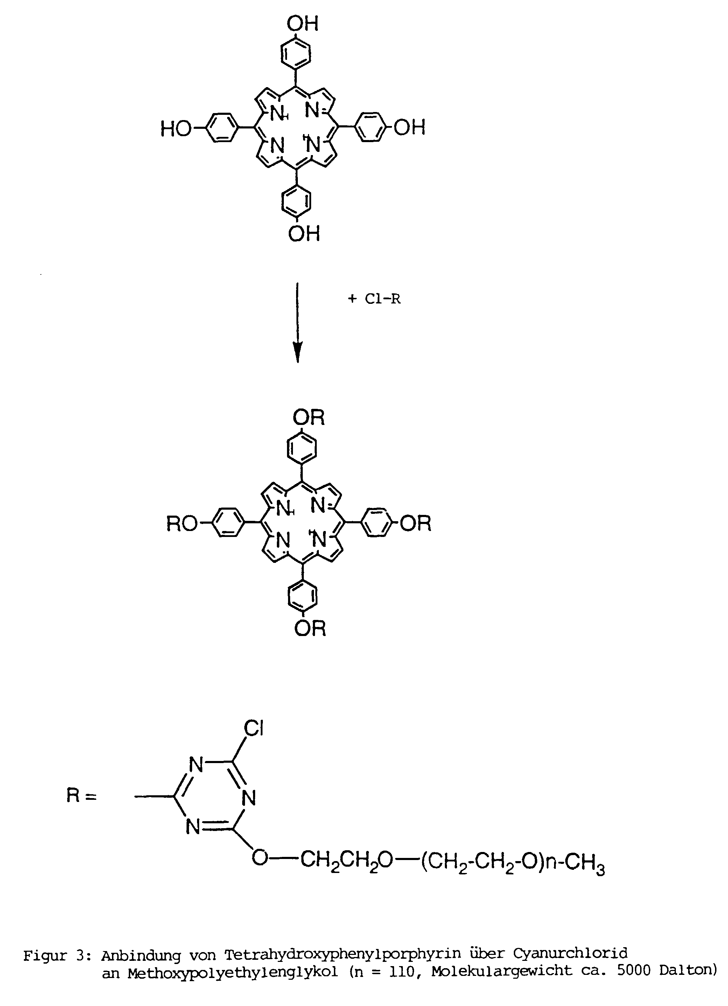

Bevorzugt verwendete Konjugate sind in den Figuren 1 bis 3 und im Beispiel angegeben.

Bezüglich weiterer Offenbarung der erfindungsgemäß verwendeten Konjugate wird auf die vorstehend genannten DE-A 39 12 792 und DE-A 40 17 439 ausdrücklich verwiesen.

Desweiteren können erfindungsgemäß verwendete Konjugate nach üblichen Verfahren hergestellt werden, durch die der Wirkstoff, der Linker, das Protein und ggf. der Polyalkohol miteinander verbunden werden. Beispielhaft wird hierzu auf die Herstellung der Konjugate der Figuren 1 bis 3 verwiesen.

Erfindungsgemäß verwendete Konjugate zeichnen sich durch eine erhöhte Halbwertszeit im Organismus aus, die zum Teil durch die geringe Ausscheidung bewirkt wird. Weiterhin reichern sich die Konjugate in Gewebe an, das von entzündlichen Erkrankungen, wie Infektions- und/oder Autoimmun- und/oder Hauterkrankungen und/oder entzündlichen Neovaskularisationen, geschädigt ist. Somit sind sie zur Therapie und/oder Diagnose von entzündlichen Erkrankungen, wie Infektions- und/oder Autoimmun- und/oder Hauterkrankungen und/oder entzündlichen Neovaskulariosationen, bestens geeignet.

Kurze Beschreibung der Zeichnung:
- Figur 1:: zeigt die Anbindung von radioaktiv markiertem Tyramin-N-1'-Desoxysorbitol (TDS) über Cyanurchlorid an Albumin.
- Figur 2:: zeigt die Anbindung von Tetracarboxyphenylporphyrin (TCPP) an Albumin,
- Figur 3:: zeigt die Anbindung von Tetrahydroxyphenylporphyrin (THOPP) über Cyanurchlorid an Methoxypolyehtylenglykol (MPEG) und
- Figur 4:: zeigt die Anreicherung von Konjugaten in entzündlichem Gewebe.

Das folgende Beispiel erläutert die Erfindung.

### Beispiel: Anreicherung von Konjugaten in entzündlichem Gewebe

In Ratten wurde durch Injektion von 2 ml Sephadexkügelchen (Sephadex G-200 Sigma Chemicals) eine Entzündung am linken Hinterbein ausgelöst. Anschließend wurde jeweils ein nachstehendes Konjugat einer Ratte verabreicht. Als Konjugate wurden verwendet:

| | |
|---|---|
| TDS-CMPEG | (CMPEG: Cyanurchlorid-aktiviertes MPEG (n = 110)), |
| THOPP-CMPEG | (vgl. Fig. 3), |
| TDS-THOPP-CMPEG | (Konjugat, bei dem an THOPP sowohl CMPEG (n = 110) als auch TDS gebunden ist), |
| TDS-Albumin | (vgl. Fig. 1), |
| TCPP-Albumin | (vgl. Fig. 2) oder |
| TDS-TCPP-Albumin | (Konjugat, bei dem an Albumin sowohl TDS über Cyanurchlorid als auch TCPP gebunden ist). |

Als Kontrolle wurde Ratten statt der Sephadexkügelchen 2 ml physiologische Kochsalzlösung injiziert und danach eines der vorstehenden Konjugate appliziert.

Die Anreicherung der Konjugate wurde über 72 Studen szintigraphisch in üblicher Weise dokumentiert.

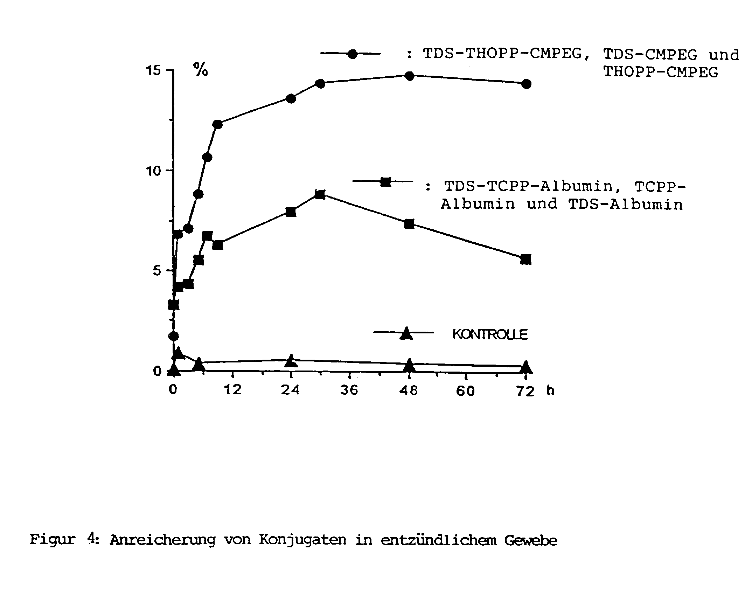

Wie aus Fig. 4 zu ersehen ist, reichern sich die Konjugate im Vergleich zur Kontrolle in entzündlichem Gewebe an.

## Patentansprüche

1. Verwendung eines Konjugates, umfassend einen Wirkstoff zur Therapie und/oder Diagnose von entzundlichen Erkrankungen, einen Linker und einen Träger ausgewählt aus Albumin, Transferrin und/oder einem Polyethylenglykol, zur Herstellung eines Arzneimittels zur Behandlung und/oder Diagnose von entzündlichen Erkrankungen.

2. Verwendung nach Anspruch 1, wobei der Wirkstoff eine radioaktiv markierte aromatische Verbindung, eine photodynamisch-aktive Verbindung und/oder ein Chemotherapeutikum ist.

3. Verwendung nach Anspruch 1 oder 2, wobei das Polyethylenglykol Methoxypolyethylenglykol ist.

4. Verwendung nach Anspruch 1 bis 3, wobei das Konjugat einen Polyalkohol aufweist.

5. Verwendung nach Anspruch 4, wobei der Polyalkohol folgende Struktur aufweist: 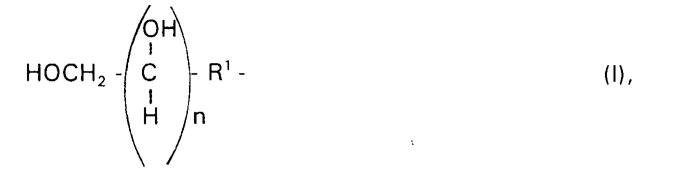 in der R¹ CHOH, CH₂, C = O oder CH₂NH und
n mindestens 1 ist.

6. Verwendung nach einem der Ansprüche 1 bis 5, wobei die entzündliche Erkrankung eine Infektions- und/oder Autoimmun- und/oder Hauterkrankung und/oder entzündliche Neovaskularisation ist.

## Claims

1. Use of a conjugate, comprising an active substance for the therapy and/or diagnosis of inflammatory diseases, a linker and a carrier selected from the group consisting of albumin, transferrin and/or a polyethylene glycol, for the production of a pharmaceutical preparation for treating and/or diagnosing inflammatory diseases.

2. Use according to claim 1, wherein the active substance is a radioactively labeled aromatic compound, a photodynamically active compound and/or a chemotherapeutic agent.

3. Use according to claim 1 or 2, wherein the polyethylene glycol is methoxypolyethylene glycol.

4. Use according to claims 1 to 3, wherein the conjugate comprises a polyalcohol

5. Use according to claim 4, wherein the polyalcohol has the following structure: 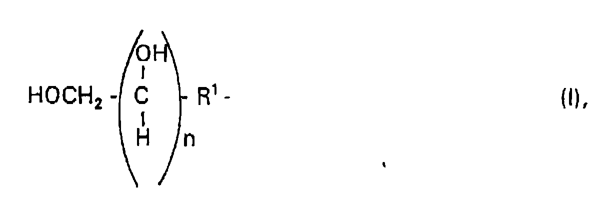 in which R¹ is CHOH, CH₂, C=O or CH₂NH and
n is at least 1.

6. Use according to any of claims 1 to 5, wherein the inflammatory disease is an infectious and/or autoimmune and/or skin disease and/or inflammatory neovascularization.

## Revendications

1. Utilisation d'un conjugué comprenant un principe actif pour le traitement et/ou le diagnostic de maladies inflammatoires, un segment de liaison et un véhicule choisi parmi l'albumine, la transferrine et/ou un polyéthylèneglycol, pour la fabrication d'un médicament pour le traitement et/ou le diagnostic de maladies inflammatoires.

2. Utilisation selon la revendication 1, le principe actif étant un composé aromatique marqué radioactivement, un composé à action photodynamique et/ou une substance chimiothérapeutique.

3. Utilisation selon la revendication 1 ou 2, le polyéthylèneglycol étant un méthoxypolyéthylèneglycol.

4. Utilisation selon les revendications 1 à 3, le conjugué comprenant un polyol.

5. Utilisation selon la revendication 4, le polyol présentant la structure suivante : 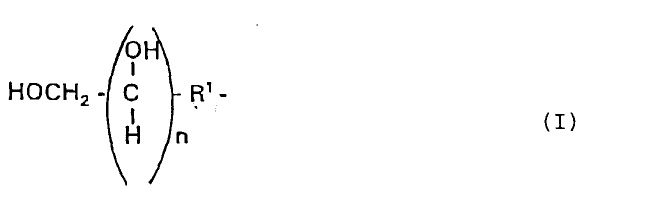 dans laquelle R¹ représente CHOH, CH₂, C=O ou CH₂NH, et
n vaut au moins 1.

6. Utilisation selon l'une des revendications 1 à 5, la maladie inflammatoire étant une maladie infectieuse et/ou auto-immune et/ou cutanée et/ou une néovascularisation inflammatoire.
